# EUROPEAN PATENT SPECIFICATION

(11) **EP 0 459 978 B1**
(45) Date of publication and mention of the grant of the patent: **16.07.1997**
(21) Application number: 89907001.5
(22) Date of filing: 03.03.1989
(51) Int. Cl.: C07K 14/74, A61K 38/08, A61K 38/10, A61K 38/16

(54) **CLASS I MHC MODULATION OF SURFACE RECEPTOR ACTIVITY**
KLASSE I-MHC-MODULATION VON OBERFLÄCHENREZEPTORAKTIVITÄT
MODULATION PAR LE COMPLEXE MAJEUR D'HISTOCOMPATIBILITE (MHC) DE CLASSE I DE L'ACTIVITE DU RECEPTEUR DE SURFACE

(43) Date of publication of application: 11.12.1991
(73) Proprietor: THE REGENTS OF THE UNIVERSITY OF CALIFORNIA, Berkeley, California 94720 (US)
(72) Inventor: GODENOW, Robert, S., Coto de Caza, CALIFORNIA 92705 (US); OLSSON, Lennart, DK-2820 Gentofte (DK)
(74) Representative: Ahner, Francis
(86) International application number: US8900876
(87) International publication number: WO9010016

(56) References cited:
- PROCEEDINGS OF THE NATIONAL ACADEMY OF SCIENCES OF USA. vol. 83, no. 16, August 1986, WASHINGTON DC, USA pages 6007 - 6011; C. DUE ET AL.: 'The major histocompatibility complex class I heavy chain as a structural subunit of the human cell membrane insulin receptor: Implications for the range of biological functions of histocompatibility antigens.'
- JOURNAL OF IMMUNOLOGY. vol. 137, no. 7, 1 October 1986, BALTIMORE MD, USA pages 2293 - 2298; M. SAMSON ET AL.
- PROCEEDINGS OF THE NATIONAL ACADEMY OF SCIENCES OF USA. vol. 82, no. 24, December 1985, WASHINGTON DC, USA pages 8634 - 8637; M. FEHLMANN ET AL.: 'Molecular association between major histocompatibility complex class I antigens and insulin receptors in mouse liver membranes.'
- PROCEEDINGS OF THE NATIONAL ACADEMY OF SCIENCES OF USA. vol. 86, no. 9, May 1989, WASHINGTON DC, USA pages 3123 - 3126; T. HANSEN ET AL.: 'Inhibition of insulin receptor phosphorylation by peptides derived from major histocompatibility complex class I antigens.'
- BHAGIRATH SINGH, The Journal of Immunology, 137, No. 7, 1986 "Alloantigenic Sites on Class I Major Histocompatibility complex antigens: 61-69 Region in the First Domain of the H-2k Molecule Induces Specific Antibody and T cell responses", See the Abstract on page 2311.
- ALISON FINNEGEN, Journal of Experimental Medicine, 164, 1986 "The T Cell Repertoire For Recognition of A Phylogenetically Distant Protein Antigen" SeeTable IV and the discussion on pages 905-908
- MORTEN SIMONSEN, Progress In Allergy, 36, 1985 "Compound Receptors In Cell Membrane: Rumination from the Borderland of Immunology and Physiology" pages 151-176.

## Description

### Technical Field

The field of the subject invention concerns modulation of surface membrane responses.

### Background

The major histocompatibility complex (MHC) Class I antigens are expressed on virtually all types of vertebrate cells examined. These highly polymorphic transmembraneous glycoproteins have a 45 kD heavy chain consisting of a short cytoplasmic C-terminal tail, a transmembraneous region, and an extracellular N-terminal sequence which encompasses three domains, α₁, α₂, and α₃. The α₁- and α₂-domains carry all the immunological polymorphism, while the membrane-proximal α₃ is non-covalently associated with the 12 kD β₂ microglobulin.

The MHC Class I antigen plays an essential role in restriction of the target cell repertoire of cytotoxic T-lymphocytes (CTL), which involves preferential utilization of the different polymorphic MHC Class I antigens, H-2K, -D or -L (for mouse) or HLA-A, -B, or -C (for human), e.g. in recognition of viral infected cells. For the most part, attention has been directed to the role of the MHC Class I antigens in restricting T-cell activity. However, some authors have suggested a broader role for the antigens, which will be discussed below.

### Relevant Literature

For a review of biological functions of MHC Class I antigens see Ohno, Immunol. Rev. (1977) 33:59-69; and Simonsen, Prog. Allergy (1985) 36:151-176. For a description of the insulin receptor see Cuatrecasas, Biol. Chem. (1972) 247:1980-1991; Kasuga et al., ibid. (1982) 257:10392-10399; and Kasuga et al., ibid. (1983) 258:10973-10980. For suggestion that Class I antigens and insulin receptors interact, see Olsson, In Cell Fusion: Gene Transfer and Transformation (eds. Beers & Bassett) 395-403 (Raven Press, New York, 1984); Simonsen and Olsson, Ann. Immunol. (1983) 134D:85-92. A relationship between receptors and their specific ligands, where the reverse complement of limited regions in receptor mRNA are identified in the ligand DNA sequence is described by Bost et al., Proc. Natl. Acad. Sci. USA (1984) 82:1372-1375; and Bost et al., Biochem. Biophys. Res. Commun. (1985) 128:1373-1380. Other evidence supporting the interaction between MHC products and insulin receptor may be found in Fehlman et al., Proc. Natl. Acad. Sci. USA (1985) 82:8634-8637; Philips et al., ibid. (1986) 83:3474-3478; Due et al., ibid. (1986) 83:6007-6011, and Samson et al., J. Immunology (1986) 137:2293-2298. Suggestions of a correlation between overexpression of certain Class I products and increased metastatic potential of particular tumors may be found in Wallich et al., Nature (1985) 315:301-305; Katzav et al., Int. J. Cancer (1984) 33:47-415; Olsson, Cancer Rev. (1986) 3:91-114; and Goodenow et al., Science (1985) 230:777-783.

### SUMMARY OF THE INVENTION

Methods and compositions are provided for modulating activity of cell surface receptors. The methods employ up or down regulation of cellular production of MHC Class I antigens or employ agonist or antagonist substances for non-covalent binding to the cellular receptor or MHC Class I antigen to affect the complexation between the MHC Class I antigen and the cellular receptor or mimic the effect of the Class I antigen. The methods and compositions may be used in diagnosis and therapy.

### DESCRIPTION OF SPECIFIC EMBODIMENTS

Methods and compositions are provided for modulating the response of cell surface receptors by varying the interaction between the cell surface receptor and MHC Class I antigen. The variation may be as a result of up or down regulation of Class I antigen production or concentration at the surface, providing agonists or antagonists for mimicking the non-covalent binding of the Class I antigen to the receptor or inhibiting such binding. Modulation of the Class I antigen-receptor interaction can be used in diagnosing and treating a large variety of conditions associated with cellular membrane receptors.

Human MHC Class I antigens are HLA-A, B, C, Qa and Tl. Of particular interest involved with the modulation of cellular receptors are the HLA-B and -C antigens, particularly the α₁- and α₂-domains, more particularly the α₁-domain.

Of particular interest are the amino acid sequences involved in the polymorphic regions of α₁ and α₂, ranging from amino acid 50 to amino acid 90, more particularly amino acids 55 to 90, usually 60 to 90, more particularly 65 to 90 or 90 to 120, more usually 90 to 116, where the amino acid sequences of interest are usually in the C-terminus of the α₁-domain and N-terminus of the α₂-domain. The region 60-85, more particularly 65 to 85 or 70 to 85 are found to be of particular interest.

It is found that the amino acids from 83 to 85 may be of particular significance. For both MHC Class I D and K, or analogous HLA-B or C, the sequence is R Y Y. Peptides of particular interest will comprise this sequence and may include at least about 20, usually at least about 15, and preferably not more than about 10 amino acids on either side of the sequence, preferably having at least 5 amino acids at the N-terminal side, and more preferably not having more than about 5-amino acids at the C-terminal side. The presence of two tyrosines is particularly desirable for the insulin receptor.

Desirably, the total number of amino acids will not exceed 20, preferably not exceed about 18, more preferably not exceed about 15 with the sequences indicated above.

Also of interest is the region from about amino acid 30 to amino acid 45, more particularly 32 to 40, particularly an oligopeptide of at least four amino acids, more usually at least about six amino acids, and preferably at least about eight amino acids, where the sequence includes a tetramer involving an acidic amino acid and a basic amino acid separated by one neutral amino acid, particularly a neutral amino acid of at least five carbon atoms and one of the acidic or basic amino acids is flanked by a neutral amino acid. Of particular interest is where the intervening neutral amino acid is an aromatic or aliphatic hydrocarbon amino acid, e.g. glycine or phenylalanine.

A large number of surface membrane proteins are involved with transduction of signals and serve as receptors for a wide variety of ligands. For the most part, receptors are defined by the ligand which activates the receptor for transduction or serves to endocytose the ligand. These receptors include endocrine, paracrine and autocrine receptors, adrenergic receptors, lipoprotein receptors, opiate receptors, and steroid receptors. These receptors include surface protein receptors for asialoglycoprotein, insulin, somatostatin, somatotropins; growth factors, such as growth hormone, platelet derived growth factor, insulin like growth factor, epidermal growth factor, α-transforming growth factor, nerve growth factor, fibroblast growth factor, somatomedin, vasopressin, prostaglandins, eosinophil chemotactic factor, acetylcholine, thyroxine (TSH), epinephrine; endorphins, enkephalins and dynorphin; neurotensin, oxytocin, transferrin, substance P, lymphokines, such as 1-, 2-, 3-, and 4-, etc.; colony stimulating factors, such as GM-CFS, M-CFS, E-CFS, etc.; lipoproteins, such as low density lipoprotein; steroids, such as estrogen, androgen, glucocorticoids, corticosteroids, etc. Of particular interest are receptors which are cycled, that is, internalyzed into the cytoplasm and then returned to the plasma membrane surface. Illustrative of these receptors are the receptors for insulin, EGF, LDL, transferrin, interleukins, and asialoglycoprotein.

Modulation of the MHC Class I antigen activation can be achieved in a variety of ways. The number of MHC antigen molecules at the surface can be increased or decreased by employing compounds which activate or inhibit the Class I antigen production. These compounds include interferon, dimethyl sulfoxide (DMSO), tetradecylphorbyl acetate (TPA), and retinoic acid. Alternatively, one may modulate the complex formation by employing antibodies to the α₁- or α₂-domain, particularly the α₁-domain, which inhibit the interaction between the Class I antigen and the receptor. Either polyclonal or monoclonal antibodies may be employed, particularly monoclonal. Alternatively, one may employ the monoclonal antibodies specific for the α₁-domain to be used as immunogens for the production of anti-idiotype antibodies, which will mimic the conformation of the Class I antigen epitope to which the monoclonal antibody binds. Thus, the anti-idiotype may act as a substitute Class I antigen and may serve to block autoimmunity. The whole antibodies need not be employed, the variable region sufficing, or larger fragments such as Fab or F(ab')₂, Fab', etc.

The antibodies may be prepared in accordance with conventional ways. Particularly, the Class I antigen may be used as an immunogen and injected into an appropriate host, conveniently a mouse, for initiating an immune response. One or more booster injections may be employed at two or more week intervals. Two to three days after the last injection, the animal host may be sacrificed, the spleen isolated, and the B-lymphocytes immortalized. Various techniques exist for immortalization, conveniently fusion with a myeloid cell, followed by selecting for hybridomas and screening, under limiting dilution conditions, for hybridomas producing antibodies having the desired characteristics. Thus, in the present situation, the Class I antigen could be used for screening or the antibody to the domain of interest, in the case of the anti-idiotype.

Instead of employing antibodies, oligopeptides may be employed which are capable of mimicking the site of the Class I antigen associated with binding to the receptor or the receptor site which binds to the Class I antigen. Thus, by preparing oligopeptides having a sequence substantially conforming to a sequence of the binding domain of the Class I antigen, or active fragment thereof, one can substitute for the presence of the Class I antigen by using the oligopeptide for activation of the receptor. By modifying the sequence, for example by substitutions, deletions or insertions, where usually from 1 to 3, usually from 1 to 2, amino acids are involved, enhanced binding of the peptide to the receptor may be achieved.

By non-conservative substitutions are intended those substitutions which substantially differ as to polarity and/or size, where each of the lines in the following table indicates what are conservative substitutions.

**Table A**

| | |
|---|---|
| Neutral | |
| Aliphatic | |
| Non-polar | |
| small | G, A (P) |
| large | V, I, L |
| Polar | |
| Oxy or Thio | S, T, C, M |
| Amide | N, Q |
| Aromatic | F, W, H, Y |
| Charged | |
| Acidic | D, E |
| Basic | K, R |
| ( ) intends that the amino acid will normally not be used as a substitute for others on the same line. | |

It is found that the peptides which bind to the receptors enhance receptor activity. While not wishing to be bound to the theory, it appears that the peptides are involved with inhibiting internalization of the receptor. In this manner, the lifetime of the ligand-receptor complex is extended, so that one observes an enhanced activity as a result of binding of ligand to the receptor. In addition, there may be other effects of the peptide, such as allosteric effects, which may enhance binding affinity, activation effects, where the peptide results in activation of the receptor, so as to provide for transduction of a signal into the cytoplasm, or other effect, where the sum total of the result is an enhanced effect as compared to the absence of the peptide or MHC binding to the receptor.

In a variety of disease states, disease is as a result of a reduced presence of a particular receptor at the surface or lower affinity for the ligand. In this situation, one could reduce the density of the Class I MHC antigen or provide for the peptide at an appropriate concentration, which allows for activation of the receptor. Conditions such as diabetes, Graves disease, arthritis, ankylosing spondylitis, Reiter's disease, pain, analgesia, viral disease, etc., could be associated with inadequate receptor response.

Alternatively, in other situations, one might wish to down regulate receptor binding, where one wishes to diminish the receptor response. Illustrative of such conditions is neoplasia, arthritis, lupus erythematosus, etc., where it is desirable to reduce the response to growth factors or other secreted factors which encourage proliferation or other undesirable response. In this situation, one could treat the target cells with a drug which would enhance the population of Class I antigens at the surface.

The subject peptides may affect one activity of the receptor differently from a different activity. For example, while with the insulin receptor, glucose uptake is enhanced, the tyrosine kinase activity is diminished. Thus, the subject peptides may selectively modify a receptor having a plurality of activities.

Instead of a change in the MHC Class I antigen population at the surface, the effective concentration of Class I antigen for complexing with receptors may be reduced. It is noted that viral infections deplete Class I antigens at the surface and in appropriate situations may be used for this purpose. As already indicated, Class I antigen depletion could also be achieved using antibodies or oligopeptides which bind to the Class I antigen at or near the complexing site inhibiting complexation with the receptor or bind to the receptor at or near the complexing site inhibiting complexation with the Class I antigen. These compounds can be prepared by employing sequences comparable to polymorphic sequences, particularly in the α₁-domain of the Class I antigen, more particularly HLA-B or -C antigens (mouse H-2L or D).

Of particular interest are oligopeptides comprising at least a portion of one of the following sequences, where the oligopeptides comprise as the active sequence at least eight amino acids, more usually at least 12 amino acids, and fewer than 40 amino acids, more usually fewer than 30 amino acids, preferably, not more than about 25 amino acids, preferably being from about 8 to 25 amino acids, more preferably about 8 to 20 amino acids. It is understood that up to five, more usually up to about three substitutions or deletions may be made in the subject sequences, where the change will not be more than about 20 number %, usually not more than about 10 number % of the number of amino acids in the active sequence. Also the following sequences may be joined together either contiguously or by bridges of not more than about 20 amino acids, more usually not more than about 10 amino acids. Furthermore, where the sequnces overlap, it is intended that the overlapping sequences not be repeated, but rather the non-overlapping sequences joined in proper sequence.

The oligopeptide will have at least eight amino acids which are the same or substantially the same as a sequence included in the following sequence.
1. D T aa³² F V R F D S D aa⁴⁰ aa⁴¹
2. F V R F D S D aa⁴⁰ aa⁴¹ S P R aa⁴⁵
3. W aa⁵² E Q aa⁵⁵ aa⁵⁶ G P E Y W
4. W aa⁶¹ aa⁶² aa⁶³ T aa⁶⁵ aa⁶⁶ aa⁶⁷ K aa⁶⁹ aa⁷⁰ aa⁷¹
5. W aa⁶¹ aa⁶² aa⁶³ aa⁶⁴ aa⁶⁵ aa⁶⁶ aa⁶⁷ K aa⁶⁹ aa⁷⁰ aa⁷¹ aa⁷² aa⁷³ aa⁷⁴ aa⁷⁵ aa⁷⁶ aa⁷⁷ aa⁷⁸ aa⁷⁹ aa⁸⁰ aa⁸¹ aa⁸² aa⁸³ aa⁸⁴ aa⁸⁵
6. E Q aa⁷³ aa⁷⁴ R V aa⁷⁷ aa⁷⁸ R aa⁸⁰ aa⁸¹ aa⁸² R Y Y
wherein:
aa³² is any neutral aliphatic amino acid of from 4 to 6 carbon atoms particulary N, Q, V, I, or L, more particularly Q or L;
aa⁴⁰ is an aliphatic amino acid, charged or uncharged, usually non-polar or acidic of from 2 to 5, more usually 2 to 4 carbon atoms, particularly G, A, P, D or E, more particularly A or D;
aa⁴¹ is an aliphatic amino acid, charged or uncharged of from 2 to 5, usually 3 t 5 carbon atoms, particularly G, A, P, S, T, D or E, more particularly A, T and E;
aa⁴⁴ is P, N, or Q, particularly P or Q;
aa⁴⁵ is any aliphatic amino acid, particularly G, A, S, T, M, K, R, or E, particularly G, E, or K;
aa⁵² is a neutral aliphatic amino acid of from 4 to 6 carbon atoms, particularly V, I, L or M, more particularly V or I;
aa⁵⁵ is any charged amino acid, particularly K, R, D, or E, more particularly K or E;
aa⁵⁶ is a charged amino acid, particularly D, E, K or R, more particularly E or K;
aa⁶¹ is D or E;
aa⁶² is K, R, G, or A, particularly R or G;
aa⁶³ is any aliphatic amino acid other than basic of from 4 to 6 carbon atoms, particularly D, E, I, L, V, N, or Q, more particularly E, N, or Q;
aa⁶⁴ is S, T, or M, particularly T;
aa⁶⁵ is any polar or basic amino acid of 4 to 6 carbon atoms, particularly N, Q, K or R, more particularly Q;
aa⁶⁶ is any aliphatic amino acid of from 4 to 6 carbon atoms, particularly L, I, V, K, R, N, or Q, more particularly K, I or N;
aa⁶⁷ is any neutral aliphatic or aromatic amino acid, particularly G, A, L, V, I, S, T, M, C F, Y, N, or Q, more particularly C, S, Y, or M;
aa⁶⁸ is K or R, particularly K;
aa⁶⁹ is any aliphatic neutral or acidic amino acid, particularly D, E, G, A, S, T, or M, particularly A or T;
aa⁷⁰ is any aliphatic amino acid, neutral, polar, or basic (other than acidic) from 3 to 6, usually 4 to 6 carbon atoms, particularly N, Q, K, R, S, or T, more particularly N, Q, or K;
aa⁷¹ is any aliphatic amino acid other than basic, usually from 2 to 5 carbon atoms, particularly G, A, S, T, D, or E, more particularly A or T;
aa⁷² is N or Q, particularly Q;
aa⁷³ us S, T, F, Y, H, or W, particularly T;
aa⁷⁴ is D, E, F, Y, H, or W, particularly Y or D;
aa⁷⁵ is K or R, particularly R;
aa⁷⁶ is an aliphatic amino acid other than basic of from 4 to 6 carbon atoms, particularly D, E, V, I, or L, more particularly E or V;
aa⁷⁷ is a polar aliphatic amino acid of from 3 to 6 carbon atoms particularly N, Q, S, T, D, or E, more particularly N, D or S;
aa⁷⁸ is a non-polar aliphatic amino acid of from 3 to 6 carbon atoms, particularly A, P, V, I, or L, more particularly L;
aa⁷⁹ is K or R, particularly R;
aa⁸⁰ is a neutral aliphatic amino acid of from 3 to 6, usually 4 to 6 carbon atoms, particularly S, T, N, Q, I, V, or L, more particularly N, T, or I;
aa⁸¹ is an aliphatic non-polar amino acid, particularly G, A, L, I, or V, more particularly A or L;
aa⁸² is an aliphatic amino acid other than acidic, of from 2 to 6, usually 5 to 6, carbon atoms, particularly K, R, G, A, L, I, or V, more particularly L or R;
aa⁸³ is an aliphatic amino acid other than acidic of from 2 to 6 carbon atoms, particularly K, R, G, A, L, I, or V, more particularly G or R;
aa⁸⁴⁻⁸⁵ are aromatic amino acids, particularly F, Y, H, or W, more particularly Y.

Preferably, there will usually not be more than three mutations in the above sequence as substitutions, deletions, or insertions.

Of particular interest is an amino acid sequence of at least 6, usually at least 8, amino acids coming within the following sequence.
W D/E R aa⁶³ T Q/R aa⁶⁶ aa⁶⁷ K aa⁶⁹ aa⁷⁰ aa⁷¹ Q T/W aa⁷⁴ R V/E aa⁷⁷ L R aa⁸⁰ L/A L/R G/R Y Y wherein:
aa⁶³ is E, I, or N;
aa⁶⁶ is I, N, or K, particularly I;
aa⁶⁷ is A, C, S, M, or Y, particularly Y or C;
aa⁶⁹ is G, A, T, or P, particularly A or T;
aa⁷⁰ is Q, N, or K;
aa⁷¹ is A, E, or T;
aa⁷⁴ is D, F, or Y, particularly D or Y;
aa⁷⁷ is N, S, or D;
aa⁸⁰ is I, N, or T.
where when two amino acids are indicated at a particular site, either amino acid may be employed interchangeably. Up to three of the amino acids may be subject to conservative or non-conservative changes, there being from 0 to 2 deletions or insertions of from 1 to 2 amino acids.

The oligopeptides may be provided in a variety of ways, being joined to non-wild-type flanking regions, as fused proteins, joined by linking groups or directly covalently linked through cystine (disulfide) or peptide linkages. The oligopeptides may be joined to a single amino acid at the N- or C-terminus or a chain of amino acids. The fused peptides may be extended to provide convenient linking sites, e.g. cysteine or lysine, to enhance stability, to bind to particular receptors, ease of purification, change the physical characteristics, e.g. solubility, charge, etc, stabilize the conformation, etc. The oligopeptide may be N- or C-terminal or internal.

The oligopeptide may be joined to other peptides for a variety of purposes. The oligopeptide may be linked through a variety of bifunctional agents, such as maleimidobenzoic, methyldithioacetic acid, mercaptobenzoic acid, S-pyridyl dithiopropionate, etc. The oligopeptides may be linked to proteins to provide immunogens for the production of antibodies. The oligopeptides may be linked, particularly by an intracellular cleavable linkage, to antibodies for site directed action. The oligopeptides may be linked to peptides to enhance stability, for site directed action, to provide additional physiological activity or the like. For conjugation techniques, see, for example, U.S. Patent Nos. 3,817,837; 3,853,914; 3,850,752; 3,905,654; 4,156,081; 4,069,105; and 4,043,989, which are incorporated herein by reference.

The oligopeptides may also be modified by incorporation into the lumen of vesicles, e.g. liposomes, which in turn may be bound to ligands or receptors for direction to particular cells or tissue.

The oligopeptides may be used as ligands to determine the presence of particular receptors as a diagnostic. Thus, cells could be screened, intact or as a lysate, for the population of one or more receptors which bind the oligopeptide. The oligopeptides could be labeled, directly or indirectly, with a label which provides a detectable signal, e.g. radioisotope, fluorescers, enzyme, particle, chemiluminescer, etc. Thus, cells from tissue, e.g. biopsies, blood, or the like may be diagnosed in vitro or in vivo for the presence of receptors binding to the oligopeptides. In addition, the binding pattern with the MHC Class I antigen of various cells can be determined. Diseased states as a result of inadequate complexation between MHC Class I antigens and receptors can be determined. A large number of protocols are known and have been developed and appear in the patent and scientific literature. Commercially available assays include ELISA, EMIT, SLFIA, RIA, etc.

The oligopeptides may be employed in a variety of ways. For therapy, they may be administered parenterally, e.g. by injection at a particular site, for example, subcutaneously, intraperitoneally, intravascularly, or the like.

The formulations will usually involve a physiologically acceptable medium, such as deionized water, saline, aqueous ethanol, ethanol, phosphate buffered saline, and the like. Other additives may be included, such as buffers, stabilizers, other proteins, bacteriocides, or the like. The manner of formulation will vary depending upon the purpose of the formulation, the particular mode employed for modulating the receptor activity, the intended treatment, and the like. The formulation may involve capsules, liposomes, time delayed coatings, pills, or be formulated in pumps for continuous administration. Because of the wide variety of modes of treatment, the varying responses, the different disease states, or the like, no useful limits may be given for the concentration of the active components. These can be determined empirically in accordance with known ways. See, for example Harrison's, Principles of Internal Medicine, 11th ed. Braunwald et al. ed, McGraw Hill Book Co., New York, 1987.

The oligopeptides of this invention may be prepared in accordance with conventional techniques, such as synthesis, recombinant techniques, or the like. See, for example, Maniatis et al., Molecular Cloning: a laboratory manual, CSH Laboratory, Cold Spring Harbor, New York, 1982; use of a Beckman Model 990 peptide synthesizer or other commercial synthesizer.

The following examples are offered by way of illustration and not by way of limitation.

### EXPERIMENTAL

Mutant line R1E, derived from the murine thymoma line R1 by chemical mutagenesis (Parns and Seidman, Cell (1982) 29:661-669; Allen et al., Proc. Natl. Acad. Sci. USA (1986) 83:7447-7451) expresses in contrast to R1, none of the parental H2^{k} haplotype antigens of the C58 strain of origin due to the lesions induced in the β₂-microglobulin (β2m) gene in R1E. Specific insulin binding to R1E murine thymoma cells and various R1E transfectants was determined, with the binding being performed as described in Gavin et al., J. Biol. Chem. (1973) 248:2202-2207 and Due et al., Diabetologia (1985) 28:749-755.

All cells were cultivated in RPMI-1640 with 15% fetal calf serum (FCS) and with the various additives as indicated (Allen et al., supra (1986)). Prior to insulin binding assays, the cells were seeded in RPMI-1640 with 10% FCS and at a density of 2 x 10⁴ cells/ml, harvested three days later, viability assured to be >95% by trypan dye exclusion, and the cells subsequently resuspended in assay buffer for insulin binding at a concentration of 7.5 x 10⁷ cells/ml. ¹²⁵I-labeled human insulin in a final concentration of 50 pM (labeled in the A14 position and obtained from NOVO A/S, Denmark) was added and the cells incubated for 90 minutes at 18°C in a shaking waterbath. Two ml ice-cold assay buffer were added at the end of incubation, the cells centrifuged at 300 g for 5 minutes, at 100 g for 10 minutes, and the amount of ¹²⁵I-insulin in the pellet counted in a γ-counter. Non-specific binding was estimated as the amount of ¹²⁵I-insulin binding in the presence of 10⁻⁶M unlabeled insulin, and specific insulin binding calculated as the difference in binding of ¹²⁵I-insulin with and without unlabeled insulin. Specific binding <1% was estimated to be non-specific considering the Scatchard plots and the specific binding as related to cell number.

Scatchard plots were done for lines R1, R1E, R1E/β2m, R1E/D^{b}, R1E/β2m/K^{b}, R1E/β₂M/D^{b}, R1E/β2m/D^{b}Δ, and R1E/β2m/D^{b} - (1 + 2) with 3 x 10⁷ cells per sample and each point representing duplicate or triplicate samples. The Scatchard plots were repeated 3-10 times for each determination. Only R1 and R1E/β2m/D^{b} displayed applicable amounts of insulin receptor (IR). The curve observed shows that in addition to high affinity IR, these cells also have appreciable amounts of receptors with lower affinity for insulin, which may to some extent be due to indirect effects of transfection and/or co-expression of other insulin binding receptors such as those for IGF-I (Rechler and Hessley In Polypeptide Hormone Receptors (ed B.I. Posner) pp 227-297, Marcel Dekker, New York (1985)).

The R1 murine thymoma cells have a cell surface density of IR comparable to other lymphocyte cell populations in contrast to the human IM-9 cell line often used for insulin assays, and which is an Epstein-Barr virus transformed line with exceptionally high amounts of non-functional IR. It was accordingly necessary in the R1/R1E system to use comparatively high amounts of cells per sample. Titration of specific insulin binding as related to cell number demonstrated that the optimal cell number per sample for specific insulin binding was 7 x 10⁷ cells - impractical to use on all Scatchard plots. The curves for R1 and R1E/β2m/K^{b} show that these two lines did not express significant amounts of IR.

Insulin receptor mRNA in R1, R1E and R1E transfectants was determined as follows. Total RNA was isolated from cells as per Chirgwin et al., Biochemistry (1979) 18:5294-5299, and poly A+ RNA selected as per Maniatis et al., Molecular Cloning 1A Laboratory Manual CSH Laboratory, Cold Spring Harbor, New York, (1982). For Northern blot analysis, 5 µg of poly A+ selected murine liver mRNA was fractionated on a 1.0% agarose-formaldehyde gel (Church and Gilbert, Proc. Natl. Acad. Sci. USA (1984) 81:1991-1995) and blotted on a Zeta-probe nylon membrane (Bio Rad Laboratories, Richmond, CA). Insulin receptor-specific sequences were detected by hybridization with a synthetic DNA oligonucleotide representing amino acids 732-741 inferred from the insulin receptor cDNA precursor (Ullrich, et al., Nature (1985) 313:756-761). Hybridization and washing conditions were as per Church and Gilbert, supra (1984), except that hybridization and washes were at 45°C. Approximately 1 µg and 1:10 dilution of poly A+ selected mRNA from mouse liver and appropriate cell lines was spotted on the Zeta-Probe membranes and hybridized as above. Molecular weight markers for Northern blot analysis were purchased from Bethesda Research Laboratories (Bethesda, MD). A predominant species of 4.8 kb from mouse liver hybridized to the human insulin receptor oligonucleotide. This species was also noted by Ullrich et al., supra (1985), in human placental mRNA with radiolabeled cloned human insulin receptor cDNA sequences.

Reverse complementarity between D^{k}α1 and insulin receptor was determined as follows. The reverse (3'-5') complement of the murine MHC Class I D^{k} and K^{k} genes were screened for nucleic acid and protein sequence using DNASIS (Hitachi Corp., Japan, and Intelligenetics, Palo Alto, CA) computer programs. 75% amino acid homology was noted between the reverse complement of K^{k}α1 aa 34-41 and the human insulin receptor signal peptide aa 14-22. All noted homologies between D^{k} and K^{k} and human insulin, estrogen, epidermal growth factor, or interleukin-2 receptors were considerably lower (<50%). The degree of homology between D^{k}α1 and IR is within the limits of homology considered significant by Bost et al., Biochem. Biophys. Res. Commun. (1985) 128:1373-1380.

Figure 3

N term
C term
D^k aa 34-41 --- Arg Phe Asp Ser Asp Ala Glu Asn ---

5'
3'
D^k nt 100-125 --- GCGC TTC GAC AGC GAC GCG GAG AAT ---

↓

3'
5'
D^k complement --- CGCG AAG CTG TCG CTG CGC CTC TTA ---

Deduced peptide
from reverse Arg Glu Ala Val Ala Ala Pro Leu
(3'-5') . . . . . . . .
complement . . . . . .

Insulin receptor --- Arg Gly Ala Ala Ala Ala Pro Leu ---
signal sequence
aa-22 - -14

The two amino acids neglecting to show exact homology between the reverse complement of D^{k}α1 and the human IR are mismatches in the third base codon. Exact homology is represented by two ·, whereas third base mismatch is represented by one ·. By scanning 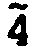 000 available sequences in the Bionet gene bank, no significant homology was detected between the deduced peptide or its DNA equivalent.

The surface proteins of the various cell lines were screened using fluorescent labeled monoclonal antibodies and a FACS.

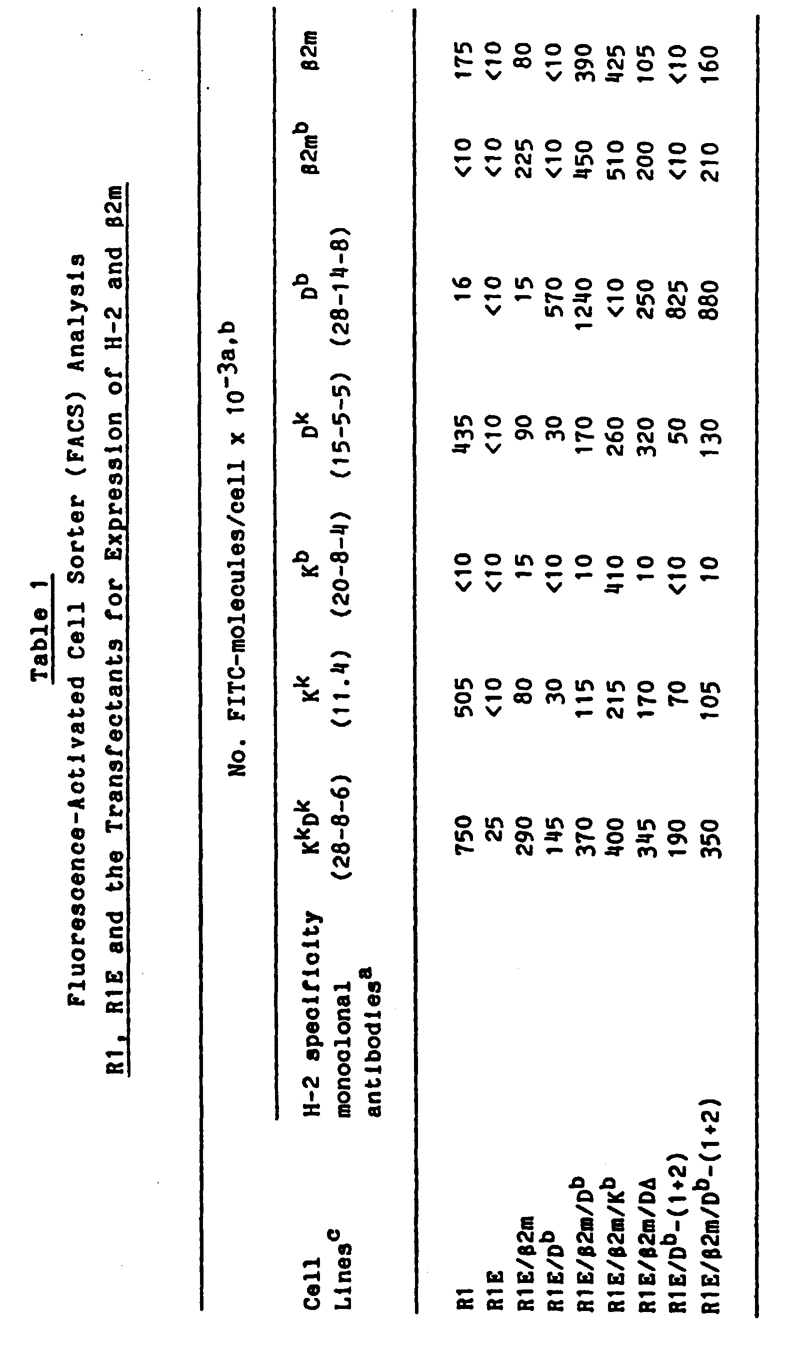

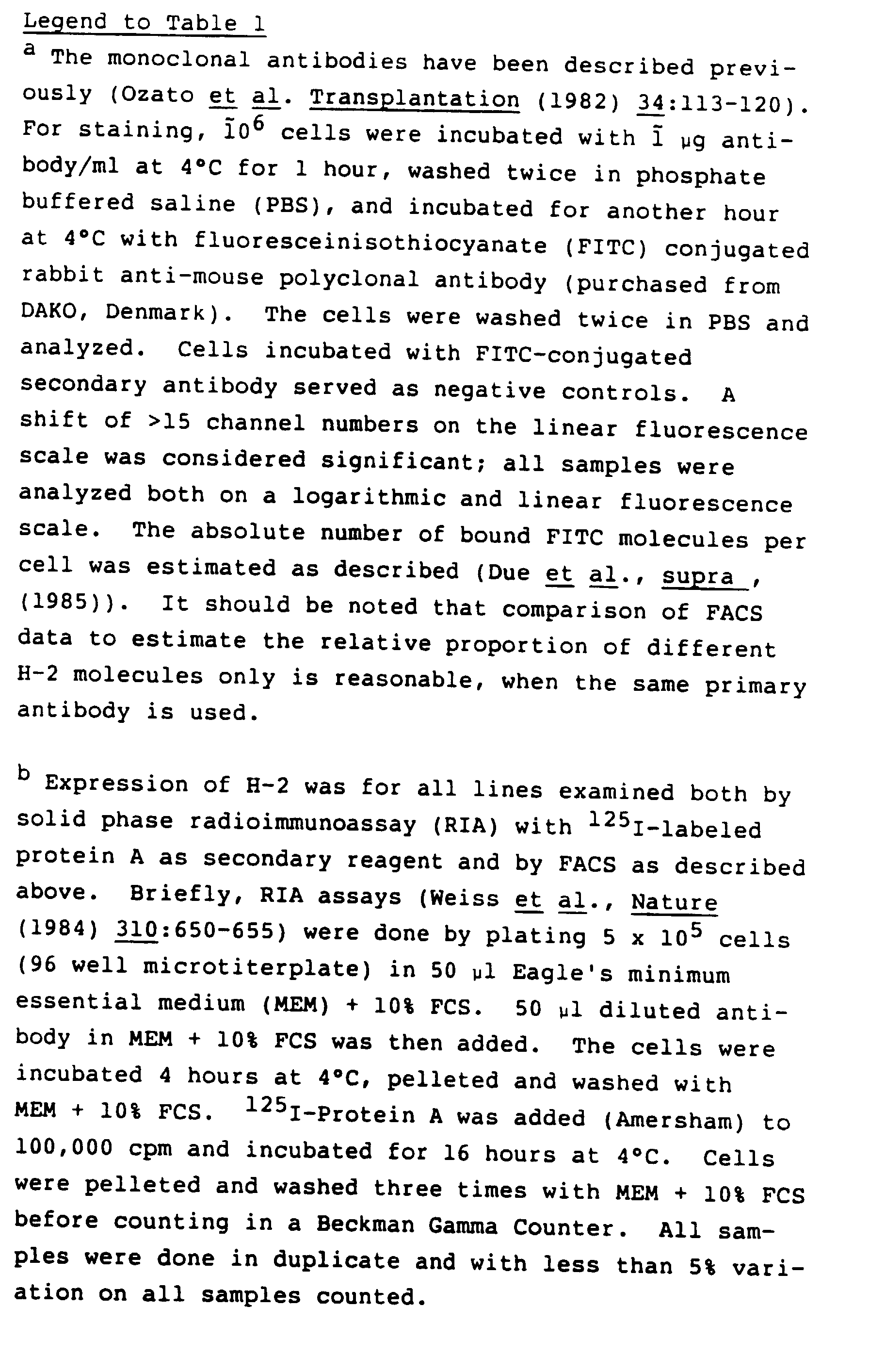

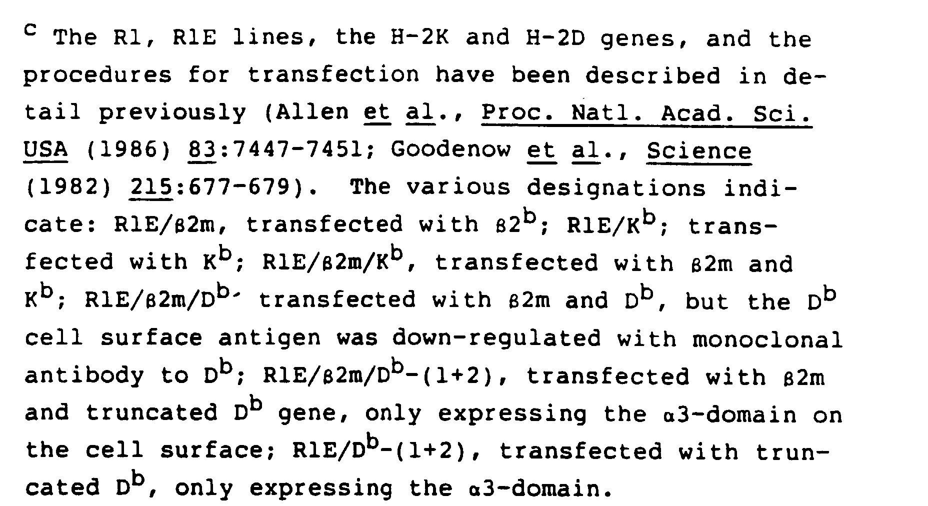

Adenocarcinoma cell line LT85 (Callahan et al., J. Immunol. (1983) 471-474) lacks appreciable cell surface expression of the H-2 antigens characteristic of the strain of derivation. Interferon-α (IFN-α) treatment significantly increases the cell surface density of both H-2K^{k}, H-2D^{k} and increases in parallel the specific binding of insulin. Alternatively, the ultraviolet light-induced fibrosarcoma LR335 (Daynes et al., Transplantation (1977) 23:343-348), unlike R1E or LT85, expresses appreciable levels of the H-2K^{k} antigen, endogenous to the strain of origin with negligible H-2D^{k}, unless induced with IFN. As shown in Table 2, IFN treatment markedly increases both H-2D^{k} expression and the binding of insulin, approaching the levels of the other positive cell lines tested. These results support the conclusion drawn from work on the R1E transfectants: the control of IR expression maps to the end of the MHC D region.

**Table 2**

| Effect of Interferon on MHC Class I and Insulin Receptor Expression | | | | |
|---|---|---|---|---|
| H-2 allelle Expression^{a} | | | | |
| Cell Line | Interferon Treatment | K^{k} (16-1-11) | D^{k} (15-5-5) | Specific Insulin Binding (%) |
| LT85 | None | 3,370 | 1,030 | 2.0 |
| | + | 19,170 | 6,500 | 3.2 |
| LR 335 | None | 16,230 | 1,000 | <1.0 |
| | + | 28,770 | 6,639 | 2.1 |

| | | | | |
|---|---|---|---|---|
| ^{a} Estimated by radioimmunoassay as described in Table 1 and the results given as cpm/5 x 10⁵ cells ^{b} The interferon treatment was done with alpha-interferon. | | | | |

To map the control of insulin receptor in a third haplotype, several Class I variants of the BALB/c S49 thymoma line (Joseph et al., J. Immunol. (1986) 137:4016-4020) were tested for insulin binding. As shown in Table 3, the H-2K/H-2D positive variant displayed significant hormone binding relative to the H-2K minus variant, suggesting that the D molecules support the cell surface expression of the receptor. The homology shared between H-2D^{b} and H-2L^{d} suggest that the H-2L locus also exerts an interaction with the IR.

**Table 3**

| Expression of MHC Class I and Insulin Receptor in Six Murine Cell Lines of Two Different Haplotypes | | | | | | |
|---|---|---|---|---|---|---|
| MHC Class I Expression^{b} | Cell lines^{a} | | | | | |
| | 3LL/G2 | 3LL/G4 | 28 | 33 | 36 | 29 |
| K^{b} (20-8-4) | 14,840 | 2,500 | - | - | - | - |
| D^{b} (28-14-8) | 3,130 | 7,440 | - | - | - | - |
| K^{d} (20-8-4) | - | - | 3,572 | 1,124 | 940 | 292 |
| D^{d} (34-1-2) | - | - | 4,930 | 661 | 278 | 280 |
| L^{d} (28-14-8) | - | - | 559 | 297 | 261 | 341 |
| K^{k} (negative control;11.4) | - | - | 254 | 297 | 286 | 595 |
| Specific Insulin Binding (%) | 2.4 | 5.0 | 3.1 | 1.8 | <1.0 | <1.0 |

| | | | | | | |
|---|---|---|---|---|---|---|
| ^{a} All cell lines have been described elsewhere (Olsson and Forehhammer, Proc. Natl. Acad. Sci. USA (1984) 81:3389-3393; Joseph et al., J. Immunol. (1986) 137:4016-4020). The lines numbered 28, 33, 36, and 29 have previously been designated S49.1, S49.2, S49.3, and S49.4, respectively. | | | | | | |
| ^{b} Estimated by RIA (see Table 1) with monoclonal antibodies as indicated and the data given as cpm/5 x 10⁵ cells. | | | | | | |

### Study of MHC Class I Peptide

### MATERIAL AND METHODS

### Cell Culture

All cell lines are grown in minimal essential medium (MEM, Gibco) supplemented with 2 mM glutamine, 50 U/ml penicillin, 50 µg/ml streptomycin, 1.0% non-essential amino acids, 1 mM sodium pyruvate and 10% fetal bovine serum (Hyclone) excepting R1E and R1E transfectants which are grown in RPMI (Gibco) and supplemented as above. The following cell lines are used in this study: P815- a murine mastocytoma of H-2^{d} origin; R1E and related transfectants- murine lymphomas of H-2^{k} origin but transfected with H-2^{b} genes (Allen et al., (1986)); human lymphoblastoid lines #1 and #2-Epstein-Barr virus transformed lymphocytes from two related individuals, #2 being a Type I diabetic; LR335-a UV induced murine fibrosarcoma of H-2^{k} origin.

### Hormone Binding

Hormone binding assays are based on descriptions by Freychet Diabetologia (1976) 12(2):85-100; and Freychet et al., Endocrinology (1977) 100:115-121 with modifications. All reactions are carried out in 200 µl volumes in 96-well, round-bottomed microtiter plates (Dynatech Labs, VA) in MEM (Gibco) with 0.5% bovine serum albumin (Sigma fraction V) and 0.1% sodium azide to prevent internalization and subsequent degradation of membrane proteins. Sodium azide at 0.1% does not alter insulin binding. Cells are resuspended at 1 x 10⁷ to 1 x 10⁸, depending on the cell type and, when appropriate, pre-incubated at 4° for one hour in the presence of peptide at 100 µg/ml. Specific insulin binding is measured using ¹²⁵I-insulin (Amersham, 2000 Ci/mmole, 370 kBq/10 uCi) as tracer at 50-100 pM. Incubation is carried out for 90 minutes at 20°. Cells are then washed three times in ice-cold MEM to remove unbound insulin and the pellets resuspended to 100 µl and counted in a Beckman gamma counter. Non-specific insulin binding is measured in the presence of 100 µg/ml unlabeled porcine insulin (Sigma). Specific EGF binding is measured in a similar fashion using ¹²⁵I-EGF (Amersham, 150 µCi/mg) as tracer and unlabeled mouse submaxillar gland EGF (Sigma) at 1 µg/ml is used as cold competitor.

### RESULTS

### Enhanced Insulin Binding Mediated by an MHC Class I Peptide

To investigate H-2/IR interactions on the cell surface, a peptide was synthesized corresponding to the α₁ domain, aa⁶¹⁻⁸⁵, of the H-2L^{d} protein. The sequence is E-R-I-T-Q-I-A-K-G-Q-E-Q-W-F-R-V-N-L-R-T-L-L-G-Y-Y.

When increasing amounts of this peptide were added to cells in culture, the synthetic Class I analogue significantly boosted the hormone binding on P815 mastocytoma cells in a dose-dependent fashion. Whereas the peptide was able to enhance receptor activity by at least an order of magnitude, peptides of similar size representing sequences from a T-cell receptor or MHC Class II sequences failed to augment the levels of hormone binding on P815. In addition, an H-2L^{d} peptide synthesized without glutamic at position 71, but otherwise identical to L^{d} 61-85, also was unable to affect insulin binding, demonstrating the exquisite specificity of this peptide for the receptor.

Several other cell types were tested for L^{d} peptide enhancement of insulin binding, including freshly isolated murine spleen. (See Table 4) Cells of both human and murine origin demonstrated insulin binding augmentation to varying degrees by the L^{d} peptide. The magnitude of induction on BALB/c spleen cells supports the in vivo effect of the peptide.

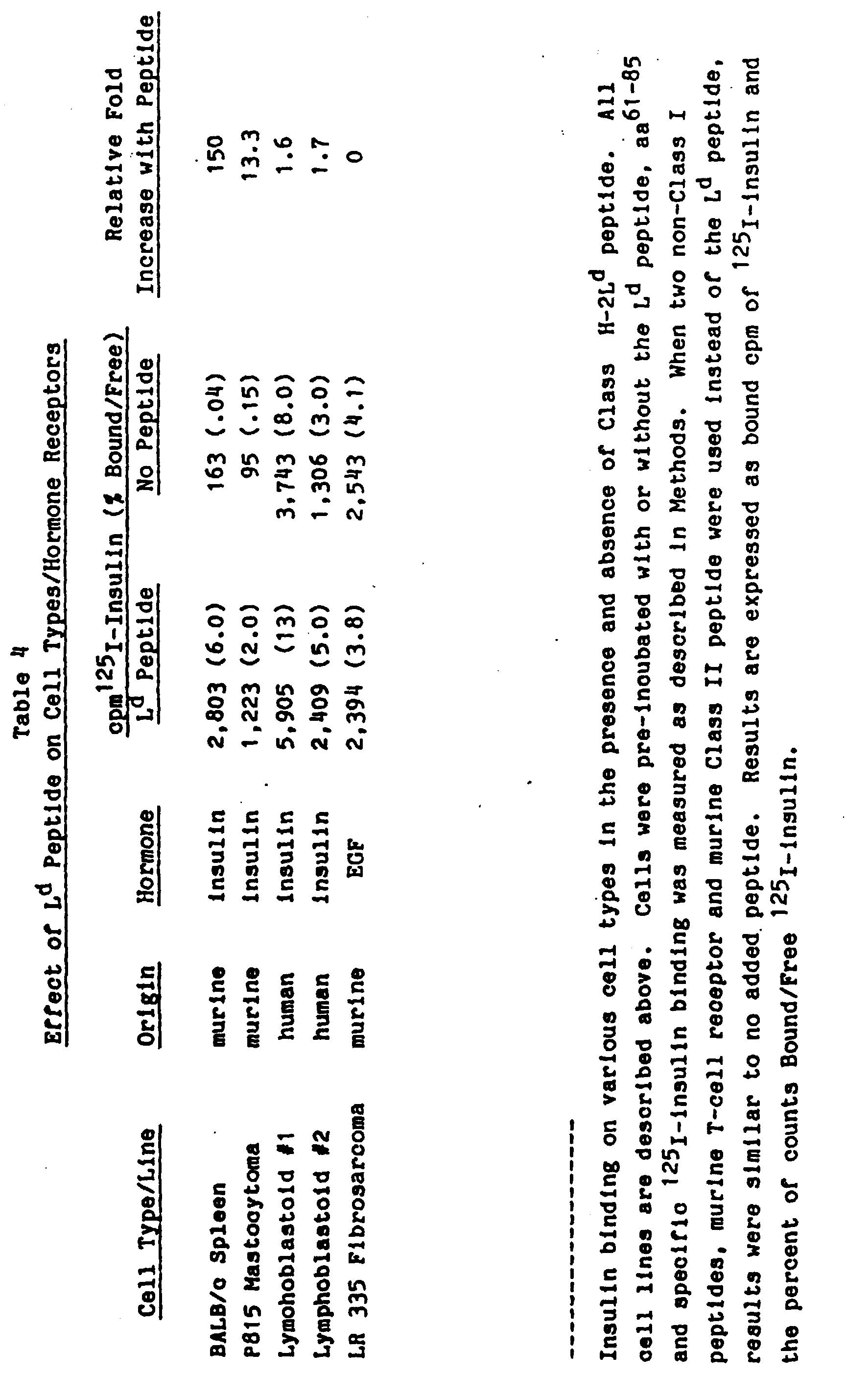

Furthermore, no effect was observed when epidermal growth factor (EGF) binding was measured on a fibrosarcoma expressing EGF receptors, showing further specificity for the peptide in interacting with insulin receptors.

Additional insulin binding studies were carried out on a series of related cells possessing different surface expression profiles of Class I/IR complexes. The insulin binding level on RIE, RIE+B21t+D^{b} were tested in the presence and absence of L^{d} peptide. The H-2L^{d} peptide was capable of augmenting the IR found on the D^{b} transfectants alone - both RIE and RIE+B2M+K^{b} failed to show appreciable L^{d} peptide induced enhancement in insulin binding.

**Table 5**

| Effect of L^{d} Peptide on Insulin Binding on R1E, R1E+β₂M+D^{b}, R1E+β₂M+K^{b} | | |
|---|---|---|
| Cell Line | cpm ¹²⁵I-Insulin (%B/F) | |
| | L^{d} Peptide | No Peptide |
| R1E | 752 (1.0) | 393 (1.0) |
| R1E + β₂M + D^{b} | 935 (2.0 | 471 (1.0) |
| R1E + β₂M + K^{b} | 478 (1.0) | 432 (1.0) |

In agreement with the previous observations, the H-2K^{d} transfectants appear to lack cell surface IR since no appreciable hormone binding could be induced with the peptide. Consequently, these data confirm that the D-end products control the transport of receptor to the cell surface.

Other studies were performed with 61-85 fragments of D^{k} antigens, as well as the use of a number of control peptides. Insulin activity was segregated into two factors, tyrosine kinase activity and glucose uptake.

### Peptides

The two MHC Class I derived peptides Dk(61-85), and K^{k}-(61-85) are both from the same region of the α1 domain of the MHC Class I molecules (Klein, Natural history of the major histocompatibility complex (Wiley, New York)). Both peptides were synthesized by Applied Biosystems, Inc., (Foster City, CA), and quality controlled by mass spectrometry.

The D^{k}-(61-85) and K^{k}-(61-85) peptides were iodinated for some experiments using carrier-free Na¹²⁵I (Amersham) and iodobeads (Pierce) by incubating for 20 min., then purified by reversed-phase HPLC on a C₁₈ column (Beckman) in a linear 30-50% gradient of CH₃CN in 5 mM trifluoroacetic acid (TFA). The ¹²⁵I-labeled peptide eluting first was stored at 4°C in 50% CH₃CN/5 mM TFA. The labeled peptides were stable under these conditions for at least 3 months.

Control Peptides: ACTH-(1-24) (human), CCK-33 (porcine), dynorphin A (porcine), β-endorphin-(1-27) (camel), glucagon (human), and prosomatostatin-(1-32) (porcine) were all purchased from Peninsula Laboratories, Belmont, CA. The A-chain and B-chain of insulin (porcine) and glucagon-(1-21) (human) were obtained from Novo Industry, Denmark. ACTH-(1-24) was used as a routine control peptide.

### Purified Insulin Receptor

The purified human IR and the cloned cytoplasmic kinase domain (IRKD) have been described (Ellis et al. (1988) Virology 62:1634-39; Roth et al. (1986) J. Biol. Chem. 261:3753-57). Briefly, the human IR was purfied from placenta by immunoaffinity columns, using monoclonal antibodies and binding of IR to wheat germ agglutinin. The product was a tetramer with two heavy chains, each ~130 kDa, and two light chains, each ~90 kDa.

### Tyrosine Kinase Activity

The cytoplasmic, cloned IRKD was constructed from the IR sequence (Ebena et al. (1985) Cell 40:747-758; Ullrich et al. (1985) Nature 313:756-761) and expressed in insect cells by using a baculovirus expression vector. The domain is soluble (Mᵣ ~48 kDa) and the kinase activity is constitutively expressed in vitro. The IRKD was purified to homogeneity by immunoaffinity chromatography.

The procedures to measure kinase activity of the purified IR and IRKD, and the effects of insulin have been described elsewhere (Roth et al. (1986) supra). Briefly, 5.0 µl purified IR was mixed with 5.0 µl insulin (final concentration 1.0 µM), and buffer (50 mM HEPES, pH 7.6, 150 mM NaCl, 0.1% Triton X-100) added to a final volume of 20 µl. When peptide was used, it was added in 5.0 µl, the volume adjusted to 20 µl by adding buffer, and the mixture incubated (1 hr, 4°C). After incubation, 10 µl of a solution containing 2.5 µCi ³²P-labeled ATP (3,000 Ci/mmol; γ-labeled; Amersham), 50 mM HEPES, pH 7.6, 150 mM NaCl, 0.1% Triton X-100, 37.5 µM unlabeled ATP, 15 mM MgCl₂, and 6 mM MnCl₂ was added to a final volume of 30 µl. The mixture was then incubated for 30 min. at 24°C.

After incubation, 15 µl sample buffer was added, and the sample was boiled for 5 min., and run on 10% SDS-PAGE overnight. The gel was dried, and autoradiograms processed with an exposure time of 5-10 hr. For quantitative estimates the β-subunit band of IR and the IRKD bands were cut out and counted dry (Cerenkov) in a scintillation counter.

Substrate phosphorylation was done with poly-([Glu,Tyr];4:1) (Sigma) as substrate. The substrate was added to a final concentration of 1.0 mg/ml and the phosphorylation assay was conducted as described above. For quantitative estimates the entire lane from just below the insulin receptor band to a 20-kDa marker was cut out and counted or the substrate was precipitated with TCA. For the latter, 5 µl sample was dotted on to 3 MM paper (Whatman), washed 30 min. in ice cold 10% TCA, boiled 10 min. in 5% TCA, then washed twice in distilled water and twice in ethanol, and finally dried and counted.

### Insulin Binding

Porcine monoiodinated [¹²⁵I]-insulin (iodinated at Tyr A14; 1,900-2,000 Ci/mmol) was obtained from NOVO Industry and Amersham. Unlabeled porcine insulin (NOVO) was dissolved in 10 mM HCl at 1 mM and stored immediately at -20°C.

The plate assay for insulin binding to its purified receptor has been described (Morgan and Roth (1985) Endocrinology 116, 1224-1226). Briefly, 50 µl of affinity-purified rabbit anti-mouse IgG (Jackson Immuno Research Lab., Inc., West Grove, PA) (40 µg/ml) in 20 mM NaHCO₃, pH 9.6, was added to 96-well polyvinyl chloride (PVC) plates. The plates were incubated (17-20 hrs, 4°C), washed thrice in 50 mM HEPES, pH 7.8, with 150 mM NaCl, 0.1% Triton X-100, 0.05% BSA, and 2 x 10⁻⁸ M monoclonal antibody (Amac, Inc., Westbrook, ME) was added. After incubation (1 hr, 24°C), the plates were washed, and insulin binding measured.

For binding measurements, ¹²⁵I-insulin (3 x 10⁻¹⁰M) was added together with increasing amounts of unlabeled insulin, and incubated (90 min., 24°C), washed, and the amount of free and bound ¹²⁵I-labeled insulin measured. Bound insulin was determined by eluting IR off the plate with 0.1M HCl and measuring in a γ-counter. For data analysis, non-specific binding was defined as the amount bound in presence of 10⁻⁶M unlabeled insulin.

### Results

The effect of D^{k}-(61-85) on both substrate (poly-[E,Y]) phosphorylation and IR autophosphorylation as a function of the peptide concentration, wherein IR tyrosine kinase activity is induced with 10⁻⁶ M insulin was determined. Both are strongly inhibited at a concentration of µM D^{k}-(61-85). The basal activity of IR (no insulin added) is inhibited 24-40% by D^{k}-(61-85) and K^{k}-(61-85). The effect of K^{k}-(61-85) is significantly weaker than D^{k}-(61-85) on autophosphoylation, with EC₅₀ values [95% confidence intervals] of 4.0 )M [2.2-7.2 )M] and 1.2 )M [0.3-2.2 )M] for K^{k}-(61-85) and D^{k}-(61-85), respectively, whereas no difference is observed in respect to substrate phosphorylation. None of the control peptides (e.g. ACTH-(1-24) are substrates for IR tyrosine kinase.

No significant depletion (degradation or adsorption), as examined by HPLC and ¹²⁵I-labeled D^{k}(61-85), K^{k}-(61-85), ACTH-(1-24), or dynorphin A is observed during the experimental period at concentrations above 0.1 )M. The D^{k}-(61-85) peptide does not affect IRKD phosphorylation, as demonstrated by pre-incubation of maximally autophosphorylated and ³²P-labeled IR for 1 hr on ice with 10 )M peptide and subsequent incubation with 500 )M cold ATP for 0-60 min. at room temperature.

The D^{k}-(61-85) has no effect on the binding of insulin to IR. The EC₅₀ IR autophosphorylation is about 3 x 10⁻⁹ M insulin, corresponding approximately to K_{d}(2.8 x 10⁻⁹M). D^{k}-(61-85), at 10 )M inhibits autophosphorylation at all insulin concentrations.

D^{k}-(61-85), 3 )M inhibits the insulin-induced IR autophosphorylation, but not the insulin receptor kinase domain phosphorylation, when IR and IRKD are used at comparable activities. IR is not a significant substrate for IRKD in the absence of insulin. IR becomes a significant substrate for IRKD when insulin is added. This observation is facilitated by the inhibitory effect of the peptide on IR autophosphorylation, because the IR phosphorylation as mediated by the tyrosine kinase of IR itself and the phosphorylation mediated by IRKD would otherwise be indistinguishable.

In the next study, the uptake of glucose in rat adipocytes was performed. Adipocytes are prepared from non-starved male rat epididymal fat pads (1.2-1.6 g fat per rat) by collagenase digestion. The buffered is KRH with 5% BSA: only plastic tubes are used. The digest is filtered (25 µ{) washed and resuspended in approximately 4 x the cell volume (estimated by lipocrit). An aliquot is removed for Coulter counting after staining with 2% osmiun tetroxide, filtration and dilution in saline. 50 µl of adipocyte suspension is added to the pre-incubation mix; 300 µl buffer, 50 µl insulin (80 nM) or buffer; 50 µl test solution (10 x) or buffer and incubated for 30 min. at 37°C in a shaking water bath. A blank without cells is included for background counting. D-[¹⁴C]-glucose is subsequently added (about 10⁵dpm/sample) and incubation continued for 60 min. The incubation is terminated by layering the 400 µl sample on top of silicone oil, followed by a 30 sec. microfuge spin, and cutting the adipocytes (thin layer of cells on top of the oil, buffer under oil) into LS vials with scintillation fluid. Glucose concentration was about 300 nM (sp.a. 295 mCi/mmol).

The effect of increasing concentrations of insulin in 30 µM D^{k}-(61-85) on glucose uptake was determined. Insulin induced maximally and 8-11 fold increase in glucose uptake as compared to basal uptake. Addition of D^{k}-(61-85) increased the maximal uptake to about 14-18 fold of basal, a glucose uptake above maximal insulin stimulation. At low concentrations of insulin (plasma level and lower), 30 µM D^{k}-(61-85) increased glucose uptake as much or more than insulin on a molar basis.

The maximal effect of D^{k}-(61-85) was obtained at 15 µM. It is found that the increase varies with the particular peptide batch, where the insulin effect of the peptide may vary from about 20% to 100%.

Various fragments of D^{k}-(61-85) were prepared by enzymatic digestion with specific peptidases: endo K, which gave fragments 61-68 and 69-85; endo E, which gave fragment 78-85; CP Y, which provided fragment 61-84; and in addition, the starting fragment was iodinated, which would be expected to occur at the termimal tyrosines. Each of the fragments were tested for biological activity after purification (greater than 95%) by HPLC and added to cells to a final concentration of 30 µM. The results reported as percent activity of the mean ± SE, with the starting fragment being 100 are as follows (61-68) 19 ± 22; (69-85) 87 ± 2; (78-85) 15 ± 3; (61-84) 19 ± 3; iodinated fragment 9 ± 10.

The effect of D^{k}-(61-85) in whole rats was determined. D^{k}-(61-85) (2.5 mg/kg) and insulin (10 µg/kg) on the blood glucose levels in rats (100-300 g) was determined. The peptide and insulin were injected i.v. after the animals had been anesthetized with pentobarbital. All animals were starved 16-20 hrs. prior to experimentation. Each determination was based on results as obtained from 42 rats, where the same rats were used in the four treatment schedules. The schedules were a control, a peptide by itself, insulin by itself, and insulin plus peptide. The control showed no significant change in blood glucose over the 240 min. during which determinations were made. The peptide, at about 20 min., the blood glucose had dropped to about 65% of its original value and then slowly rose back to about the original value at about 90 min. and was maintained about the same level. A similar result was observed with the injection of insulin. However, where the insulin and peptide were injected together, the glucose dropped within about 20 min. to about 55% of its original value and slowly rose to about 85% of its original value at about 195 min. then gradually increase to about 90% at about 240 min. Calculation of the area between the control curve and the experimental curves from T = 0 to T = 240 showed that the area for insulin plus peptide is significantly larger than that of insulin or peptide alone, indicating a prolonged hypoglycemia period as compared to the insulin or peptide alone.

In order to determine the main target organs for peptide mediated glucose, the glucose uptake in various organs was analyzed as a function of olgopeptide injection i.v. The main target organs for peptide mediated glucose uptake are skeletal muscle, liver and kidney, when the size of the organ is considered. It is notable that some of these organs are not affected by insulin injection. The procedure employed was the injection of ¹⁴C-2-deoxyglucose 60 min. after injection of insulin plus peptide and the organ content of ¹⁴C measured 30 min. later, i.e. 90 min. after injection of peptide.

Based on the above data, it may be concluded that D^{k}-(61-85) peptide enhances cellular glucose uptake both in the absence and presence of insulin. Peptide effect is increased upon stimulation with insulin. Maximal peptide effect is reached at a peptide concentration of 10-20 µM. The peptide causes enhanced glucose uptake significantly above that induced by maximal insulin stimulation. The effect in vitro is maximal after 20 min. incubation of the cells with peptide. Intravenous injection of 2.5 mg/kg D^{k}(61-85) peptide causes a decrease in blood glucose in whole animals. It is accentuated when insulin is injected together with the peptide. Glucose uptake as induced by peptide is particularly pronounced in muscle, liver and kidney, but the peptide does not result in increased levels of serum-insulin.

It is evident from the above results that surface membrane receptors involving transduction of signals, as exemplified by the insulin receptor, are modulated by MHC Class I antigens, particularly H-2D and -L of mice and HLA-B and -C of humans. A wide variety of physiological processes, both in vitro and in vivo, may be regulated by controlling the interaction between the appropriate Class I antigen and the surface membrane receptor, by a variety of techniques which allow for the enhancement or reduction of the interaction between the Class I antigen and the surface membrane receptor.

All publications and patent applications mentioned in this specification are indicative of the level of skill of those skilled in the art to which this invention pertains. All publications and patent applications are herein incorporated by reference to the same extent as if each individual publication or patent application was specifically and individually indicated to be incorporated by reference.

Although the foregoing invention has been described in some detail by way of illustration and example for purposes of clarity of understanding, it will be obvious that certain changes and modifications may be practiced within the scope of the appended claims.

## Claims

1. An oligopeptide of at least 8 amino acids comprising a sequence of the α1 domain of a major histocompatibility complex Class I HLA-B or -C antigen or mammalian equivalent thereof consisting essentially of a sequence coming within one of the following sequence units:
(a) DTaa³²FVRFDSDaa⁴⁰ aa⁴¹
(b)FVRFDSDaa⁴⁰ aa⁴¹ S P R aa⁴⁵
(c) Waa⁵²EQaa⁵⁵aa⁵⁶GPEYW
(d) W aa⁶¹ aa⁶² aa⁶³ T aa⁶⁵ aa⁶⁶ aa⁶⁷ K aa69 aa⁷⁰ aa⁷¹ Q
(e) W aa⁶¹ aa⁶² aa⁶³ aa⁶⁴ aa⁶⁵ aa⁶⁶ aa⁶⁷ K aa⁶⁹ aa ⁷⁰ aa⁷¹ aa⁷² aa⁷³ aa⁷⁴ aa ⁷⁵ aa⁷⁶ aa⁷⁷ aa⁷⁸ aa⁷⁹ aa⁸⁰ aa⁸¹ aa⁸² aa⁸³ aa⁸⁴ aa⁸⁵
f) E Qaa⁷³ aa⁷⁴ R V aa⁷⁷ aa⁷⁸ R aa⁸⁰ aa⁸¹ aa⁸² R Y Y
wherein:
aa³² is any neutral aliphatic amino acid of from 4 to 6 carbon atoms;
aa⁴⁰ is G, A, D or E;
aa⁴¹ is G, A, D or E;
aa⁴⁴ is P, N, or Q
aa⁴⁵ is any aliphatic amino acid;
aa⁵² is V, I, L or M;
aa⁵⁵ is any charged aliphatic amino acid;
aa⁵⁶ is a charged amino acid;
aa⁶¹ is D or E;
aa⁶² is K, R, G or A;
aa⁶³ is any aliphatic amino acid other than basic of from 4 to 6 carbon atoms;
aa⁶⁴ is S, T or M;
aa⁶⁵ is any polar or basic amino acid of from 4 to 6 carbon atoms;
aa⁶⁶ is any aliphatic amino acid of from 4 to 6 carbon atoms;
aa⁶⁷ is any neutral aliphatic or aromatic amino acid;
aa⁶⁸ is K or R;
aa⁶⁹ is any aliphatic neutral amino acid;
aa⁷⁰ is any aliphatic amino acid other than acidic of from 3 to 6 carbon atoms;
aa⁷¹ is any aliphatic amino acid other than basic;
aa⁷² is N or Q;
aa⁷³ is S, T, F, Y, H or W;
aa⁷⁴ is D, E, F, Y, H or W;
aa⁷⁵ is K or R;
aa⁷⁶ is an aliphatic amino acid other than basic of from 4 to 6 carbon atoms;
aa⁷⁷ is a polar aliphatic amino acid of from 3 to 6 carbon atoms;
aa⁷⁸ is a neutral aliphatic amino acid of from 5 to 6 carbon atoms;
aa⁷⁹ is K or R;
aa⁸⁰ is a neutral aliphatic amino acid of from 3 to 6 carbon atoms;
aa⁸¹ is a neutral aliphatic non-polar amino acid;
aa⁸² is a aliphatic amino acid other than acidic;
aa⁸³ is an aliphatic amino acid other than acidic;
aa⁸⁴ and aa⁸⁵ are aromatic amino acids;
with the proviso . that there are not more than three amino acid mutations as deletions, insertions or substitutions, and . that said oligopeptide having the sequence FRETQKAKG is excluded.

2. An oligopeptide according to Claim 1, wherein said oligopeptide sequence comes within the following unit:
W D/E R aa⁶³ T Q/R aa⁶⁶ aa⁶⁷ K aa⁶⁹ aa⁷⁰ aa ⁷¹ QT/W aa⁷⁴ R V/E aa⁷⁷ L R aa⁸⁰ L/A L/R G/R Y Y, wherein:
aa⁶³ is E, I, or N;
aa⁶⁶ is I, N, or K, particularly I;
aa⁶⁷ is A, C, S, M, or Y, particularly Y or C;
aa⁶⁹ is G, A, T, or P, particularly A or T;
aa⁷⁰ is Q N, or K;
aa⁷¹ is A, E, or T;
aa⁷⁴ is D, F, or Y, particularly D or Y;
aa⁷⁷ is N, S, or D;
aa⁸⁰ is I, N, or T,
where aa/aa intends either amino acid may be present.

3. An oligopeptide according to Claim 1, wherein said oligopeptide sequence comes within the following unit:
W D/E R aa⁶³ T Q/R aa⁶⁶ aa⁶⁷ K aa⁶⁹ aa⁷⁰ aa⁷¹ QT/W aa⁷⁴ R V/E aa⁷⁷ L R aa⁸⁰ L/A L/R G/R Y Y, wherein:
aa⁶³ is E, I, or N;
aa⁶⁶ is I, N, or K, particularly I;
aa⁶⁷ is A, C, S, M, or Y, particularly Y or C;
aa69 is G, A, T, or P, particularly A or T;
aa⁷⁰ is Q N, or K;
aa⁷¹ is A, E, or T;
aa⁷⁴ is D, F, or Y, particularly D or Y;
aa⁷⁷ is N, S, or D;
aa⁸⁰ is I, N, or T,
where aa/aa intends either amino acid may be present,
with the proviso that there are no more than three amino acid mutations as deletions insertions or substitutions and at least one amino acid of the terminal sequence RYY is present.

4. A peptide conjugate comprising an oligopeptide according to Claim 1 covalently bonded to at least one amino acid, wherein said amino acid is other than the wild type amino acid.

5. A peptide conjugate according to Claim 4, wherein said at least one amino acid is an immunogenic polypeptide capable of stimulating an immune response in a vertebrate host.

6. A method for detecting the presence of an MHC Class I antigen dependent receptor present on a cell surface or at least a portion of a lysate, said method comprising:
combining a sample suspected of containing said receptor with a peptide conjugate according to Claim 2; and
determining the presence of a complex between said peptide conjugate and said receptor.

7. A method for detecting the presence of an MHC Class I antigen dependent receptor present on a cell surface or at least a portion of a lysate, said method comprising;
combining a sample suspected of containing said receptor with an oligopeptide according to Claim 1; and
determining the presence of a complex between said oligopeptide and said receptor.

8. An oligopeptide according to Claim 1 bonded to a label capable of providing directly or indirectly a detectable signal.

9. A method for regulating surface receptor response of a mammalian cell, said method comprising:
modulating the number of binding complexes or functionally equivalent binding complexes between (1) a major histocompatibility complex human Class I HLA-B or -C antigen or other mammalian equivalent thereof and (2) said surface receptor on said cell by combining with said cell an oligopeptide according to Claim 1, whereby said receptor response is modulated.

10. A method according to Claim 9, wherein said modulating is binding an oligopeptide to said antigen inhibiting the formation of said complexes.

11. A method according to Claim 9, wherein said receptor is internalised after binding to its ligand.

12. A method for regulating an endocrine surface receptor response of a human cell, said method comprising:
modulating the number of binding complexes or functionally equivalent binding complexes between (1) a major histocompatibility complex human Class I HLA-B or -C antigen and (2) said endocrine surface receptor on said cell by combining with said cell an oligopeptide according to Claim 1, whereby said receptor response is modulated.

13. A method according to Claim 12, wherein said amino acid sequence of said oligopeptide is identical to an amino acid sequence of said antigen and is of at least about 8 amino acids and includes the sequence R Y Y.

14. A method for regulating an insulin receptor response of a mammalian cell, said method comprising:
modulating the number of binding complexes or functionally equivalent binding complexes between (1) a major histocompatibility complex human Class I HLA-B or -C antigen or mammalian equivalent thereof and (2) said insulin receptor on said cell by combining with said cell an oligopeptide according to Claim 1, whereby said receptor response is modulated.

15. A method according to Claim 14, wherein said sequence is within the sequence of the amino acid sequence from amino acid 55 to 90 of the α1domain of said antigen and includes the sequence R Y Y.

16. A method according to Claim 15, wherein said amino acid sequence is the sequence from amino acid 61 to 85.

## Patentansprüche

1. Oligopeptid aus mindestens 8 Aminosäuren, eine Sequenz der α1-Domäne eines Klasse-I-HLA-B oder -C-Antigens des Haupt-Histokompatibilitätskomplexes oder ein Säugeräquivalent dieser umfassend, welche im Wesentlichen aus einer Sequenz. fallend unter eine der folgenden Sequenzeinheiten, besteht:
(a) DTAs³²FVRFDSDAS⁴⁰AS⁴¹
(b) FVRFDSDAs⁴⁰As⁴¹SPRAs⁴⁵
(c) WAs⁵²EQAs⁵⁵As⁵⁶GPEYW
(d) W As⁶¹ As⁶² As⁶³ T As⁶⁵ As⁶⁶ As⁶⁷ K As⁶⁹ As⁷⁰ As⁷¹ Q
(e) W As⁶¹ As⁶² As⁶³ As⁶⁴ As⁶⁵ As⁶⁶ As⁶⁷ K As⁶⁹ As⁷⁰ As⁷¹ As⁷² As⁷³ As⁷⁴ As⁷⁵ As⁷⁶ As⁷⁷ As⁷⁸ As⁷⁹ As⁸⁰ As⁸¹ As⁸² As⁸³ As⁸⁴ As⁸⁵
(f) E Q As⁷³ As⁷⁴ R V As" As⁷⁸ R As⁸⁰ As⁸¹ As⁸² R Y Y
wobei :
As³² eine beliebige neutrale aliphatische Aminosäure mit 4 bis 6 Kohlenstoffatomen ist;
As⁴⁰ ein G, A, D oder E ist;
As⁴¹ ein G, A, D oder E ist;
As⁴⁴ ein P, N oder Q ist;
As⁴⁵ eine beliebige aliphatische Aminosäure ist;
As⁵² ein V, I, L oder M ist;
As⁵⁵ eine beliebige geladene Aminosäure ist;
As⁵⁶ eine geladene Aminosäure ist;
As⁶¹ ein D oder E ist;
As⁶² ein K, R, G oder A ist;
As⁶³ eine beliebige aliphatische, aber keine basische, Aminosäure mit 4 bis 6 Kohlenstoffatomen ist;
As⁶⁴ ein S, T oder M ist;
As⁶⁵ eine beliebige polare oder basische Aminosäure mit 4 bis 6 Kohlenstoffatomen ist;
As⁶⁶ eine beliebige aliphatische Aminosäure mit 4 bis 6 Kohlenstoffatomen ist;
As⁶⁷ eine beliebige neutrale aliphatische oder aromatische Aminosäure ist;
As⁶⁸ ein K oder R ist;
As⁶⁹ eine beliebige aliphatische neutrale Aminosäure ist;
As⁷⁰ eine beliebige aliphatische, aber keine saure, Aminosäure mit 3 bis 6 Kohlenstoffatomen ist;
As⁷¹ eine beliebige aliphatische, aber keine basische, Aminosäure ist;
As⁷² ein N oder Q ist;
As⁷³ ein S, T, F, Y, H oder W ist;
As⁷⁴ ein D, E, F, Y, H oder W ist;
As⁷⁵ ein K oder R ist;
As⁷⁶ eine aliphatische, aber keine basische, Aminosäure mit 4 bis 6 Kohlenstoffatomen ist;
As⁷⁷ eine polare aliphatische Aminosäure mit 3 bis 6 Kohlenstoffatomen ist;
As⁷⁸ eine neutrale aliphatische Aminosäure mit 5 bis 6 Kohlenstoffatomen ist;
As⁷⁹ ein K oder R ist;
As⁸⁰ eine neutrale aliphatische Aminosäure mit 3 bis 6 Kohlenstoffatomen ist;
As⁸¹ eine neutrale aliphatische unpolare Aminosäure ist;
As⁸² eine aliphatische, aber keine saure, Aminosäure ist;
As⁸³ eine aliphatische, aber keine saure, Aminosäure ist;
As⁸⁴ und As⁸⁵ aromatische Aminosäuren sind;
mit der Bedingung, daß nicht mehr als drei Aminosäuremutationen als Deletionen, Insertionen oder Substitutionen auftreten und daß ein besagtes Oligopeptid, die Sequenz E R E T Q K A K G besitzend, ausgeschlossen ist.

2. Oligopeptid gemäß Anspruch 1, worin besagte Oligopeptidsequenz in die folgende Einheit fällt:
W D/E R As⁶³ T Q/R As⁶⁶ As⁶⁷ K As⁶⁹ As⁷⁰ As⁷¹ Q T/W As⁷⁴ R V/E As⁷⁷ L R As⁸⁰ L/A L/R G/R Y Y, wobei:
As⁶³ ein E, I oder N ist;
As⁶⁶ ein I, N oder K, insbesondere ein I ist;
As⁶⁷ ein A, C, S, M oder Y, insbesondere ein Y oder C ist;
As⁶⁹ ein G, A, T oder P, insbesondere ein A oder T ist;
As⁷⁰ ein Q, N oder K ist;
As⁷¹ ein A, E oder T ist;
As⁷⁴ ein D, F oder Y, insbesondere ein D oder Y ist;
As⁷⁷ ein N, S oder D ist;
As⁸⁰ ein I, N oder T ist,
wobei As/As bedeutet, daß die eine oder andere der beiden Aminosäuren anwesend sein kann.

3. Oligopeptid gemäß Anspruch 1, worin besagte Oligopeptidsequenz in die folgende Einheit fällt:
W D/E R As⁶³ T Q/R As⁶⁶ As⁶⁷ K As⁶⁹ As⁷⁰ As⁷¹ Q T/W As⁷⁴ R V/E As⁷⁷ L R As⁸⁰ L/A L/R G/R Y Y, wobei:
As⁶³ ein E, I oder N ist;
As⁶⁶ ein I, N oder K, insbesondere ein I ist;
As⁶⁷ ein A, C, S, M oder Y, insbesondere ein Y oder C ist;
As⁶⁹ ein G, A, T oder P, insbesondere ein A oder T ist;
As⁷⁰ ein Q, N oder K ist;
As⁷¹ ein A, E oder T ist;
As⁷⁴ ein D, F oder Y, insbesondere ein D oder Y ist;
As⁷⁷ ein N, S oder D ist;
As⁸⁰ ein I, N oder T ist,
wobei As/As bedeutet, daß die eine oder andere der beiden Aminosäuren anwesend sein kann mit der Bedingung, daß nicht mehr als drei Aminosäuremutationen als Deletionen, Insertionen oder Substitutionen auftreten, und daß mindestens eine Aminosäure der terminalen Sequenz R Y Y vorhanden ist.

4. Peptidkonjugat, umfassend ein Oligopeptid gemäß Anspruch 1, welches kovalent mit mindestens einer Aminosäure verbunden ist, wobei besagte Aminosäure eine andere, als die Aminosäure des Wildtyps ist

5. Peptidkonjugat gemäß Anspruch 4, worin die besagte mindestens eine Aminosäure ein immunogenes Polypeptid ist, welches geeignet ist eine Immunantwort in einem Wirbeltier als Wirtstier zu stimulieren.

6. Verfahren zum Nachweisen der Anwesenheit eines an der Zelloberfläche befindlichen, von einem Klasse-I-Antigen des Haupt-Histokompatibilitätskomplexes abhängigen Rezeptors oder zumindest eines Teils von einem Lysat, wobei besagtes Verfahren
das Zusammenbringen einer Probe, welche unter dem Verdacht steht den besagten Rezeptor zu enthalten mit einem Peptidkonjugat gemäß Anspruch 2; und
die Bestimmung der Anwesenheit eines Komplexes zwischen besagtem Peptidkonjugat und besagtem Rezeptor, umfaßt

7. Verfahren zum Nachweisen der Anwesenheit eines an der Zelloberfläche befindlichen, von einem Klasse-I-Antigen des Haupt-Histokompatibilitätskomplexes abhängigen Rezeptors oder zumindest eines Teils von einem Lysat, wobei besagtes Verfahren
das Zusammenbringen einer Probe, welche unter dem Verdacht steht den besagten Rezeptor zu enthalten mit einem Peptidkonjugat gemäß Anspruch 1; und
die Bestimmung der Anwesenheit eines Komplexes zwischen besagtem Peptidkonjugat und besagtem Rezeptor, umfaßt.

8. Oligopeptid gemäß Anspruch 1, welches mit einem Markierungsstoff verbunden ist, der geeignet ist ein direkt oder indirekt nachweisbares Signal zu liefern.

9. Verfahren zur Regulation der Oberflächenrezeptorantwort einer Säugerzelle, wobei besagtes Verfahren
das Modulieren der Zahl der Bindungskomplexe oder funktionell äquivalenter Bindungskomplexe zwischen
(1) einem Klasse-I-HLA-B oder -C-Antigen des Haupt-Histokompatibilitätskomplexes oder einem anderen Säugeräquivalent davon und
(2) besagtem Oberflächenrezeptor auf besagter Zelle,
durch Zusammenbringen eines Oligopeptids gemäß Anspruch 1 und besagter Zelle, wobei die besagte Rezeptorantwort moduliert wird,
umfaßt.

10. Verfahren gemäß Anspruch 9, wobei besagtes Modulieren das Binden eines Oligopeptids an das besagte Antigen und Inhibition der Bildung besagter Komplexe bedeutet.

11. Verfahren gemäß Anspruch 9, wobei besagter Rezeptor nach der Bindung an seinen Liganden internalisiert wird.

12. Verfahren zur Regulation der Antwort eines endokrinen Oberflächenrezeptors einer menschlichen Zelle, wobei besagtes Verfahren
das Modulieren der Zahl der Bindungskomplexe oder funktionell äquivalenter Bindungskomplexe zwischen
(1) einem menschlichen Klasse-I-HLA-B oder -C-Antigen des Haupt-Histokompatibilitätskomplexes und
(2) besagtem endokrinen Oberflächenrezeptor auf besagter Zelle,
durch Zusammenbringen eines Oligopeptids gemäß Anspruch 1 und besagter Zelle, wobei die besagte Rezeptorantwort moduliert wird,
umfaßt.

13. Verfahren gemäß Anspruch 12, wobei die besagte Aminosäuresequenz des besagten Oligopeptids identisch mit einer Aminosäuresequenz des besagten Antigens ist und aus mindestens 8 Aminosäuren besteht und die Sequenz R Y Y enthält.

14. Verfahren zur Regulation einer Insulinrezeptorantwort einer Säugerzelle, wobei besagtes Verfahren
das Modulieren der Zahl der Bindungskomplexe oder funktionell äquivalenter Bindungskomplexe zwischen
(1) einem menschlichen Klasse-I-HLA-B oder -C-Antigen des Haupt-Histokompatibilitätskomplexes oder einem Säugeräquivalent davon und
(2) besagtem Insulinrezeptor auf besagter Zelle,
durch Zusammenbringen eines Oligopeptids gemäß Anspruch 1 und besagter Zelle, wobei die besagte Rezeptorantwort moduliert wird,
umfaßt.

15. Verfahren gemäß Anspruch 14, wobei besagte Sequenz in der Sequenz der Aminosäuresequenz von Aminosäure 55 bis 90 der α1-Domäne des besagten Antigens liegt und die Sequenz R Y Y beinhaltet.

16. Verfahren gemäß Anspruch 15, wobei besagte Aminosäuresequenz die Sequenz von Aminosäure 61 bis 85 ist.

## Revendications

1. Oligopeptide constitué d'au moins 8 acides aminés, comprenant une séquence du domaine α1 d'un antigène HLA-B ou -C de la classe I du complexe majeur d'histocompatibilité, ou un de ses équivalents de mammifère, constitué essentiellement d'une séquence entrant dans l'une des unités de séquence suivantes :
(a) D T aa³² F V R F D S D aa⁴⁰ aa⁴¹
(b) V R F D S D aa⁴⁰ aa⁴¹ S P R aa⁴⁵
(c) W aa⁵² E Qaa⁵⁵ aa⁵⁶ G P E Y W
(d) Wa⁶¹ aa⁶² aa⁶³ T aa⁶⁵ aa⁶⁶ aa⁶⁷ Kaa⁶⁹ aa⁷⁰ aa⁷¹ Q
(e) W aa⁶¹ aa⁶² aa⁶³ aa⁶⁴ aa⁶⁵ aa⁶⁶ aa⁶⁷ K aa⁶⁹aa⁷⁰ aa⁷¹ aa⁷² aa⁷³ aa⁷⁴ aa⁷⁵ aa⁷⁶ aa⁷⁷ aa⁷⁸ aa⁷⁹ aa⁸⁰ aa⁸¹ aa⁸² aa⁸³ aa⁸⁴ aa⁸⁵
f) E Qaa⁷³ aa⁷⁴ R V aa⁷⁷ aa⁷⁸ R aa⁸⁰ aa⁸¹ aa⁸² R Y Y
où
aa³² est un acide aminé aliphatique neutre quelconque ayant de 4 à 6 atomes de carbone ;
aa⁴⁰ est G, A, D ou E ;
aa⁴¹ est G, A, D ou E ;
aa⁴⁴ est P, N ou Q ;
aa⁴⁵ est un acide aminé aliphatique quelconque ;
aa⁵² est V, I, L ou M ;
aa⁵⁵ est un acide aminé aliphatique chargé quelconque ;
aa⁵⁶ est un acide aminé chargé ;
aa⁶¹ est D ou E ;
aa⁶² est K, R, G ou A ;
aa⁶³ est un acide aminé aliphatique quelconque autre que basique ayant de 4 à 6 atomes de carbone ;
aa⁶⁴ est S, T ou M ;
aa⁶⁵ est un acide aminé polaire ou basique quelconque ayant de 4 à 6 atomes de carbone ;
aa⁶⁶ est un acide aminé aliphatique quelconque ayant de 4 à 6 atomes de carbone ;
aa⁶⁷ est un acide aminé aliphatique ou aromatique neutre quelconque ;
aa⁶⁸ est K ou R ;
aa⁶⁹ est un acide aminé neutre aliphatique quelconque ;
aa⁷⁰ est un acide aminé aliphatique quelconque autre qu'acide ayant de 3 à 6 atomes de carbone ;
aa⁷¹ est un acide aminé aliphatique quelconque autre que basique ;
aa⁷² est N ou Q ;
aa⁷³ est S, T, F, Y, H ou W ;
aa⁷⁴ est D, E, F, Y, H ou W ;
aa⁷⁵ est K ou R ;
aa⁷⁶ est un acide aminé aliphatique autre que basique ayant de 4 à 6 atomes de carbone ;
aa⁷⁷ est un acide aminé aliphatique polaire ayant de 3 à 6 atomes de carbone ;
aa⁷⁸ est un acide aminé aliphatique neutre ayant de 5 à 6 atomes de carbone ;
aa⁷⁹ est K ou R ;
aa⁸⁰ est un acide aminé aliphatique neutre ayant de 3 à 6 atomes de carbone ;
aa⁸¹ est un acide aminé non-polaire aliphatique neutre ;
aa⁸² est un acide aminé aliphatique autre qu'acide ;
aa⁸³ est un acide aminé aliphatique autre qu'acide ;
aa⁸⁴ et aa⁸⁵ sont des acides aminés aromatiques ;
du moment qu'il n'y a pas plus de trois mutations d'acides aminés sous forme de délétions, d'insertions ou de substitutions, et qu'est exclus ledit oligopeptide ayant la séquence ERETQKAKG.

2. Oligopeptide selon la revendication 1, dans lequel ladite séquence oligopeptidique entre dans l'unité suivante :
W D/E R aa⁶³ T Q/R aa⁶⁶ aa⁶⁷ K aa⁶⁹ aa⁷⁰ aa ⁷¹ QT/W aa⁷⁴ R V/E aa ⁷⁷ L R aa⁸⁰ L/A L/R G/R Y Y, dans laquelle
aa⁶³ est E, I ou N ;
aa⁶⁶ est I, N ou K, en particulier I ;
aa⁶⁷est A, C, S, M ou Y, en particulier Y ou C ;
aa⁶⁹ est G, A, T ou P, en particulier A ou T ;
aa⁷⁰ est Q, N ou K ;
aa⁷¹est A, E ou T ;
aa⁷⁴ est D, F ou Y, en particulier D ou Y ;
aa⁷⁷ est N, S ou D ;
aa⁸⁰ est I, N ou T,
où aa/aa indique que l'un ou l'autre des acides aminés peut être présent.

3. Oligopeptide selon la revendication 1, dans lequel ladite séquence oligopeptidique entre dans l'unité suivante :
W D/ER aa⁶³ T Q/R aa⁶⁶ aa⁶⁷ K aa⁶⁹ aa⁷⁰ aa⁷¹ QT/W aa⁷⁴ R V/E aa⁷⁷ L R aa⁸⁰ L/a L/R G/R Y Y, dans laquelle
aa⁶³ est E, I ou N ;
aa⁶⁶ est I, N ou K, en particulier I ;
aa⁶⁷est A, C, S, M ou Y, en particulier Y ou C ;
aa⁶⁹ est G, A, T ou P, en particulier A ou T ;
aa⁷⁰ est Q, N ou K ;
aa⁷¹ est A, E ou T ;
aa⁷⁴ est D, F ou Y, en particulier D ou Y ;
aa⁷⁷ est N, S ou D ;
aa⁸⁰ est I, N ou T,
où aa/aa indique que l'un ou l'autre des acides aminés peut être présent,
du moment qu'il n'y a pas plus de trois mutations d'acides aminés sous forme de délétions, d'insertions ou de substitutions, et qu'est présent au moins un acide aminé de la séquence terminale RYY.

4. Conjugué peptidique comprenant un oligopeptide selon la revendication 1 lié par liaison covalente à au moins un acide aminé, où ledit acide aminé est autre que l'acide aminé de type sauvage.

5. Conjugué peptidique selon la revendication 4, dans lequel ledit au moins un acide aminé est un polypeptide immunogène capable de stimuler une réponse immunitaire chez un hôte vertébré.

6. Procédé pour détecter la présence d'un récepteur dépendant d'un antigène de la classe 1 du CMH, présent sur une surface cellulaire ou sur au moins une portion d'un lysat, ledit procédé consistant :
à combiner un échantillon que l'on suspecte de contenir ledit récepteur avec un conjugué peptidique selon la revendication 2 ; et
à déterminer la présence d'un complexe entre ledit conjugué peptidique et ledit récepteur.

7. Procédé pour détecter la présence d'un récepteur dépendant de l'antigène de la classe 1 du CMH présent sur une surface cellulaire ou sur au moins une portion d'un lysat, procédé qui consiste :
à combiner un échantillon que l'on suspecte de contenir ledit récepteur avec un oligopeptide selon la revendication 1 ; et
à détecter la présence d'un complexe entre ledit oligopeptide et ledit récepteur.

8. Oligopeptide selon la revendication 1, fixé à un marqueur capable de fournir directement ou indirectement un signal détectable.

9. Procédé pour réguler la réponse des récepteurs de surface d'une cellule de mammifère, ledit procédé consistant à moduler le nombre de complexes de liaison ou de complexes de liaison à équivalence fonctionnelle entre (1) un antigène HLA-B ou -C humain de la classe 1 du complexe majeur d'histocompatibilité ou un équivalent d'un autre mammifère, et (2) ledit récepteur de surface se trouvant sur ladite cellule, par combinaison avec ladite cellule d'un oligopeptide selon la revendication 1, ce qui assure la modulation de la réponse dudit récepteur.

10. Procédé selon la revendication 9, dans lequel ladite modulation consiste à fixer un oligopeptide audit antigène inhibant la formation desdits complexes.

11. Procédé selon la revendication 9, dans lequel ledit récepteur est internalisé après liaison à son ligand.

12. Procédé pour réguler une réponse au récepteur endocrine de surface d'une cellule humaine, ledit procédé consistant à moduler le nombre de complexes de liaison ou de complexes de liaison à équivalence fonctionnelle entre (1) un antigène HLA-B ou -C humain de la classe 1 du complexe majeur d'histocompatibilité et (2) ledit récepteur endocrine de surface se trouvant sur ladite cellule, par combinaison, à ladite cellule, d'un oligopeptide selon la revendication 1, ce qui assure la modulation de la réponse dudit récepteur.

13. Procédé selon la revendication 12, dans lequel ladite séquence d'acides aminés dudit oligopeptide est identique à une séquence d'acides aminés dudit antigène et possède au moins environ 8 acides aminés et comprend la séquence R Y Y.

14. Procédé pour réguler une réponse au récepteur de l'insuline d'une cellule de mammifère, ledit procédé consistant à moduler le nombre de complexes de liaison ou de complexes de liaison à équivalence fonctionnelle entre (1) un antigène HLA-B ou -C humain de la classe 1 du complexe majeur d'histocompatibilité ou l'un de ses équivalents de mammifère, et (2) ledit récepteur de l'insuline se trouvant sur ladite cellule, par combinaison, à laquelle cellule, d'un oligopeptide selon la revendication 1, de façon à moduler ladite réponse du récepteur.

15. Procédé selon la revendication 14, dans lequel ladite séquence entre dans la séquence de la séquence d'acides aminés allant de l'acide aminé 55 à l'acide aminé 90 du domaine α1 dudit antigène et comprend la séquence R Y Y .

16. Procédé selon la revendication 15, dans lequel ladite séquence d'acides aminés est la séquence qui va de l'acide aminé 61 à l'acide aminé 85.
